(19) Europäisches Patentamt
European Patent Office
Office européen des brevets

(11) Publication number: **0 270 503 B1**

(12) **EUROPEAN PATENT SPECIFICATION**

(45) Date of publication of patent specification: **15.09.93**

(51) Int. Cl.$^5$: **A61K 31/245**

(21) Application number: **87830386.6**

(22) Date of filing: **03.11.87**

(54) **Utilization of bromure d'otilonium (DCI) for topic application in the gastrointestinal tract and relative pharmaceutical formulation appropriate for such use.**

(30) Priority: **04.11.86 IT 951486**

(43) Date of publication of application: **08.06.88 Bulletin 88/23**

(45) Publication of the grant of the patent: **15.09.93 Bulletin 93/37**

(84) Designated Contracting States: **AT BE CH DE ES FR GB GR LI LU NL SE**

(56) References cited:

**CHIR. GASTROENTER., vol. 15, no. 2, 1981; L.CAPURSO, pp. 288-294&NUM;**

**GASTROENTEROLOGIA Y HEPATOLOGIA, vol. 7, no. 7, 1984; J.M.BORDAS et al., pp. 362-368&NUM;**

**MED. CLIN., vol. 89, no. 6, 1987; J.M.BORDAS et al., pp. 230-234&NUM;**

(73) Proprietor: **A. MENARINI INDUSTRIE FARMACEUTICHE RIUNITE S.R.L.**
**Via Sette Santi 3**
**I-50131 Firenze(IT)**

(72) Inventor: **Casini, Alessandro**
**Via di Scandicci No. 91**
**I-50018 Scandicci Firenze(IT)**
Inventor: **Parti, Sandro**
**Via Venezia No. 3**
**I-52027 S. Giovanni Valdarno Arezzo(IT)**
Inventor: **Bucci, Paolo**
**Via Trebbia No. 11**
**I-52025 Montevarchi Arezzo(IT)**

(74) Representative: **Mannucci, Gianfranco, Dott.-Ing.**
**Ufficio Tecnico Ing. A. Mannucci Via della Scala 4**
**I-50123 Firenze (IT)**

EP 0 270 503 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a new pharmaceutical composition having as active principle otilonium bromide, which has the following formula:

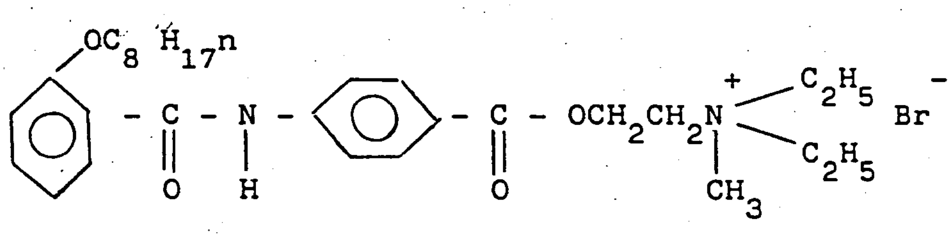

(I)

Otilonium bromide is a myolytic active substance which has been used for some time in clinical practice, by oral administration, for the therapy of spastic-dyskinetic forms of the digestive or alimentary canal both on a functional and organic basis.

In "Chir. Gastroent.", Vol.15, No.2, 1981, pages 288-294, there is described the use of otilonium bromide in premedication for endoscopic purposes. There otilonium bromide is administered orally about one hour prior to endoscopy of the upper gastrointestinal tract.

Use of otilonium bromide for colon preparation for endoscopy is described also in "Gastroenterol. y Hepatol.", Vol.7, 1984, pages 362-368. Also in this case otilonium bromide is administered by the oral route twice at time interval prior to endoscopy. The first administration took place 14 hours before endoscopy.

This known administration route has several drawbacks due, in particular, to the fact that administration must take place several hours before endoscopy is carried out. This is necessary in order to allow the otilonium bromide to reach the area where endoscopy must be carried out. Moreover, since the time required varies dramatically from subject to subject, it is quite difficult to get just the right moment to perform the endoscopy, i.e. the very moment in which the otilonium bromide becomes active in the required area.

The invention provides a new pharmaceutical form of this active principle which is suitable to effect a local application (instillation or nebulization) in the gastrointestinal tract, i.e. a topical direct administration, rather then an oral administration.

By causing an immediate stop of the motility of the gastrointestinal tract at the sprinkling point, said administration route will allow endoscopic manoeuvres both for diagnostic and therapeutic purposes (bioptic sampling, removal of polypoid formations, and others), just after the application of otilonium bromide.

According to the invention, the pharmaceutical composition comprises otilonium bromide as an active principle and an antifoaming excipient as well as an emulsifying excipient. This formulation prevents formation of foam during instillation or nebulization of the compound in the gastrointestinal tract. This allows a clear endoscopic vision of the bowel just after administration of the otilonium bromide.

The invention also refers to a kit comprising a bottle containing a pharmaceutical composition consisting of otilonium bromide as active principle and at least an antifoaming and at least an emulsifying excipient. The kit of the invention further comprises a vial of sterile water used as solvent, which is added to the content of the bottle prior to use. The solution can be used with a suitable device for instillation or nebulization inside the gastrointestinal tract.

The active principle is stored in a dry state because otilonium bromide is not stable for long time in water solution.

Basic excipients of the above pharmaceutical form can be: one or more antifoaming compounds such as linear chain primary amine salts compounds (for example tetradecylamine acetate), sulfonated oils, alkyl-phenyl-glycolethers, primary alcohols with more than five carbon atoms, polyethyleneglycols, methylic or phenylic derivatives of polysiloxane and mixtures of same; and one or more emulsifying compounds such as sugar esters (for example sucrose stearate, palmitate and others), sorbitanesters of fatty acids (such as sorbitan-monostearate - monopalmitate - monooleate and others), polyoxyethylenesorbitanesters of fatty acids (such as polyoxyethylenesorbitan-monopalmitate, -monostearate, -monooleate, and others), polyoxyethylenic esters of fatty acids (such as polyoxyetilene-stearate, -laurate, -palmitate and others), polyoxyethylenic ethers of superior alcohols (such as polyoxyethylene-laurylether, -stearylether, -cetylether, -oleylether and others), fatty acid esters (such as glycerylstearate, glycerylpalmitate, glyceryloleate, cetylpalmitate, ethyleneglycolstearate, polyethyleneglycolstearate, propyleneglycolstearate, and others).

Said formulation prevents the formation of foaming on instillation, and this advantageously assists endoscopic operations.

The preparation of the powder mixture contained in the bottle is carried out by solubilizing the excipients in an appropriate inert solvent, such as methylene chloride, chloroform, acetone, etc., in such a manner as to reach a concentration between 15% and 30%. Subsequently, said solution is made to be absorbed in a homogeneous way on the active principle utilizing a suitable apparatus. The mixture thus obtained is desiccated in an appropriate manner until the total elimination of the solvent.

Alternatively, the preparation of the above mixture can be carried out by allowing the various excipients, previously mixed and melted, to be absorbed on the active principle by using an appropriate apparatus.

The following formulations are given as unrestrictive examples:

| | | | | | | | |
|---|---|---|---|---|---|---|---|
| otilonium bromide | mg 10-300 | and | in | species | mg | 150 |
| dimethylpolysiloxane | " 0,3-10 | " | " | " | " | 5 |
| glycerylmonostearate | " 0,01-0,5 | " | " | " | " | 0,25 |
| sorbitanemonopalmitate | mg 0,08-2,5 | and | in | species | mg | 1,25 |
| sucrose monopalmitate | " 0,01-0,5 | " | " | " | " | 0,25 |
| water | ml 12-18 | " | " | " | ml | 15 |

Clinical experiments using the pharmaceutical form of the composition of the above specific example comprised the nebulization or the instillation inside the bowels (colon or stomach) or the instillation in the esophagus, before clinical examination, in subjects presenting such functional dyskinetic affections of the esophagus and of the gastroenteric system as to require an endoscopic examination for diagnostic purposes. Said endoscopic examination turned out particularly easy due to the immediate arrest of the motility of the bowel under observation, and the effectiveness of the formulation has always been satisfactory, due both to the possibility of an extempore preparation of the solution and to the presence of the antifoaming and emulsifying excipients.

## Claims

### Claims for the following Contracting States : AT, BE, CH, DE, FR, GB, IT, LI, LU, NL, SE

**1.** A pharmaceutical composition for topical use in the gastrointestinal tract, containing otilonium bromide as an active principle, characterized in that it further comprises at least an antifoaming excipient and at least an emulsifying excipient.

**2.** Pharmaceutical composition according to claim 1, characterized in that the antifoaming excipient is a methylic or phenylic derivative of polysiloxane.

**3.** Pharmaceutical composition according to claim 1 or 2, characterized in that the emulsifying excipient is chosen among one or more of the following: a sugar ester, a sorbitanester of fatty acids, a polyoxyethylenesorbitan ester of fatty acids, a polyoxyethylenic ester of fatty acids, a polyoxyethylenic ether of superior alcohols, a fatty acid ester.

**4.** Pharmaceutical composition according to any preceding claim, having the following formulation:

| | |
|---|---|
| otilonium bromide | mg 10 - 300 |
| dimethylpolysiloxane | mg 0,3 - 10 |
| glycerylmonostearate | mg 0,01 - 0,5 |
| sorbitanmonopalmitate | mg 0,08 - 2,5 |
| sucrose monopalmitate | mg 0,01 - 0,5 |
| water | ml 12 - 18 |

**5.** Pharmaceutical composition according to claim 4, having the following formulation:

| | |
|---|---|
| otilonium bromide | mg 150 |
| dimethylpolysiloxane | mg 5 |
| glycerylmonostearate | mg 0,25 |
| sorbitanmonopalmitate | mg 1,25 |
| sucrose monopalmitate | mg 0,25 |
| water | ml 15 |

**6.** A pharmaceutical kit comprising a bottle containing a pharmaceutical composition according to claim 1, and a solvent vial containing sterile water, the sterile water being added to the pharmaceutical composition prior to the topical administration.

**7.** A method for the preparation of a pharmaceutical composition according to claim 1, characterized in that the excipients are brought in solution in a solvent, the solution is absorbed on the otilonium bromide, and the solvents are removed by drying.

**8.** A method for the preparation of a pharmaceutical composition according to claim 1, characterized in that the excipients are mixed and melted, and the melted excipients are absorbed on the otilonium bromide in a suitable apparatus.

**Claims for the following Contracting States : ES, GR**

**1.** Method for the preparation of a pharmaceutical composition comprising otilonium bromide as an active principle, characterized that to said otilonium bromide there is added at least one antifoaming excipient and at least one emulsifying excipient.

**2.** Method according to claim 1, characterized in that the antifoaming excipient is a methylic or phenylic derivative of polysiloxane.

**3.** Method according to claim 1 or 2, characterized in that the emulsifying excipient is chosen among one or more of the following: a sugar ester, a sorbitanester of fatty acids, a polyoxyethylenesorbitan ester of fatty acids, a polyoxyethylenic ester of fatty acids, a polyoxyethylenic ether of superior alcohols, a fatty acid ester.

**4.** Method according to any preceding claim, characterized in that the following formulation is used:

| | |
|---|---|
| otilonium bromide | mg 10 - 300 |
| dimethylpolysiloxane | mg 0,3 - 10 |
| glycerylmonostearate | mg 0,01 - 0,5 |
| sorbitanmonopalmitate | mg 0,08 - 2,5 |
| sucrose monopalmitate | mg 0,01 - 0,5 |
| water | ml 12 - 18 |

**5.** Method according to claim 4, characterized in that the following formulation is used:

| | |
|---|---|
| otilonium bromide | mg 150 |
| dimethylpolysiloxane | mg 5 |
| glycerylmonostearate | mg 0,25 |
| sorbitanmonopalmitate | mg 1,25 |
| sucrose monopalmitate | mg 0,25 |
| water | ml 15 |

6. Method according to claim 1, characterized in that the excipients are brought in solution in a solvent, the solution is absorbed on the otilonium bromide, and the solvents are removed by drying.

7. Method according to claim 1, characterized in that the excipients are mixed and melted, and the melted excipients are absorbed on the otilonium bromide in a suitable apparatus.

**Patentansprüche**
**Patentansprüche für folgende Vertragsstaaten : AT, BE, CH, DE, FR, GB, IT, LI, LU, NL, SE**

1. Pharmazeutische Zusammensetzung zur topischen Anwendung im Gastrointestinaltrakt mit einem Gehalt an Otiloniumbromid als Wirkstoff, dadurch gekennzeichnet, daß sie weiterhin mindestens einen Antischaum-Exzipienten und mindestens einen emulgierenden Exzipienten enthält.

2. Pharmazeutische Zusammensetzung nach Anspruch 1, dadurch gekennzeichnet, daß der Antischaum-Exzipient ein Methyl- oder Phenylderivat eines Polysiloxans ist.

3. Pharmazeutische Zusammensetzung nach Anspruch 1 oder 2, dadurch gekennzeichnet, daß der emulgierende Exzipient unter einem oder mehreren der folgenden ausgewählt ist: ein Zuckerester, ein Sorbitanester von Fettsäuren, ein Polyoxyethylensorbitanester von Fettsäuren, ein Polyoxyethylenester von Fettsäuren, ein Polyoxyethylenether von höheren Alkoholen, ein Fettsäureester.

4. Pharmazeutische Zusammensetzung gemäß einem jeden der obigen Ansprüche mit der folgenden Formulierung:

| | |
|---|---|
| Otiloniumbromid | 10 - 300 mg |
| Dimethylpolysiloxan | 0,3 - 10 mg |
| Glycerylmonostearat | 0,01 - 0,5 mg |
| Sorbitanmonopalmitat | 0,08 - 2,5 mg |
| Sucrosemonopalmitat | 0,01 - 0,5 mg |
| Wasser | 12 - 18 ml |

5. Pharmazeutische Zusammensetzung gemäß Anspruch 4 mit der folgenden Formulierung:

| | |
|---|---|
| Otiloniumbromid | 150 mg |
| Dimethylpolysiloxan | 5 mg |
| Glycerylmonostearat | 0,25 mg |
| Sorbitanmonopalmitat | 1,25 mg |
| Sucrosemonopalmitat | 0,25 mg |
| Wasser | 15 ml |

6. Pharmazeutischer Kit mit einer eine pharmazeutische Zusammensetzung gemäß Anspruch 1 enthaltenden Flasche und einer steriles Wasser enthaltenden Lösemittelphiole, wobei das sterile Wasser der pharmazeutischen Zusammensetzung vor der topischen Verabreichung zugesetzt wird.

7. Verfahren zur Herstellung einer pharmazeutischen Zusammensetzung nach Anspruch 1, dadurch gekennzeichnet, daß die Exzipienten in einem Lösemittel in Lösung gebracht, die Lösung auf dem Otiloniumbromid absorbiert und die Lösemittel durch Trocknen entfernt werden.

8. Verfahren zur Herstellung einer pharmazeutischen Zusammensetzung nach Anspruch 1, dadurch gekennzeichnet, daß die Exzipienten gemischt und geschmolzen und die geschmolzenen Exzipienten auf dem Otiloniumbromid in einer geeigneten Apparatur absorbiert werden.

**Patentansprüche für folgende Vertragsstaaten : ES, GR**

1. Verfahren zur Herstellung einer pharmazeutischen Zusammensetzung mit einem Gehalt an Otiloniumbromid als Wirkstoff, dadurch gekennzeichnet, daß man zu dem genannten Otiloniumbromid mindestens einen Antischaum-Exzipienten und mindestens einen emulgierenden Exzipienten gibt.

2. Verfahren nach Anspruch 1, dadurch gekennzeichnet, daß der Antischaum-Exzipient ein Methyl- oder Phenylderivat eines Polysiloxans ist.

3. Verfahren nach Anspruch 1 oder 2, dadurch gekennzeichnet, daß der emulgierende Exzipient unter einem oder mehreren der folgenden ausgewählt ist: ein Zuckerester, ein Sorbitanester von Fettsäuren, ein Polyoxyethylensorbitanester von Fettsäuren, ein Polyoxyethylenester von Fettsäuren, ein Polyoxyethylenether von höheren Alkoholen, ein Fettsäureester.

4. Verfahren gemäß einem jeden der obigen Ansprüche, dadurch gekennzeichnet, daß die folgende Formulierung verwendet wird:

| | |
|---|---|
| Otiloniumbromid | 10 - 300 mg |
| Dimethylpolysiloxan | 0,3 - 10 mg |
| Glycerylmonostearat | 0,01 - 0,5 mg |
| Sorbitanmonopalmitat | 0,08 - 2,5 mg |
| Sucrosemonopalmitat | 0,01 - 0,5 mg |
| Wasser | 12 - 18 ml |

5. Verfahren nach Anspruch 4, dadurch gekennzeichnet, daß die folgende Formulierung verwendet wird:

| | |
|---|---|
| Otiloniumbromid | 150 mg |
| Dimethylpolysiloxan | 5 mg |
| Glycerylmonostearat | 0,25 mg |
| Sorbitanmonopalmitat | 1,25 mg |
| Sucrosemonopalmitat | 0,25 mg |
| Wasser | 15 ml |

6. Verfahren nach Anspruch 1, dadurch gekennzeichnet, daß die Exzipienten in einem Lösemittel in Lösung gebracht, die Lösung auf dem Otiloniumbromid absorbiert und die Lösemittel durch Trocknen entfernt werden.

7. Verfahren nach Anspruch 1, dadurch gekennzeichnet, daß die Exzipienten gemischt und geschmolzen und die geschmolzenen Exzipienten auf dem Otiloniumbromid in einer geeigneten Apparatur absorbiert werden.

**Revendications**
**Revendications pour les Etats contractants suivants : AT, BE, CH, DE, FR, GB, LI, LU, NL, SE**

1. Une composition pharmaceutique, pour l'utilisation locale dans les voies gastro-intestinales, contenant du bromure d'otilonium comme principe actif, caractérisée en ce qu'elle comprend de plus au moins un excipient antimousse et au moins un excipient émulsifiant.

2. Composition pharmaceutique selon la revendication 1, caractérisée en ce que l'excipient antimousse est un dérivé méthylique ou phénylique de polysiloxane.

3. Composition pharmaceutique selon la revendication 1 ou 2, caractérisée en ce que l'excipient émulsifiant est choisi parmi un ou plusieurs des suivants : un ester de sucre, un ester de sorbitan d'acides gras, un ester de polyoxyéthylènesorbitan d'acides gras, un ester polyoxyéthylénique d'acides

gras, un èther polyoxyéthylénique d'alcools supérieurs, un ester d'acide gras.

**4.** Composition pharmaceutique selon l'une quelconque des revendications précédentes ayant la formulation suivante :

| | |
|---|---|
| bromure d'otilonium | 10-300 mg |
| diméthylpolysiloxane | 0,3-10 mg |
| monostéarate de glycéryle | 0,01-0,5 mg |
| monopalmitate de sorbitan | 0,08-2,5 mg |
| monopalmitate de saccharose | 0,01-0,5 mg |
| eau | 12-18 ml |

**5.** Composition pharmaceutique selon la revendication 4 ayant la formulation suivante :

| | |
|---|---|
| bromure d'otilonium | 150 mg |
| diméthylpolysiloxane | 5 mg |
| monostéarate de glycéryle | 0,25 mg |
| monopalmitate de sorbitan | 1,25 mg |
| monopalmitate de saccharose | 0,25 mg |
| eau | 15 ml |

**6.** Un nécessaire pharmaceutique comprenant une bouteille contenant une composition pharmaceutique selon la revendication 1 et un flacon de solvant contenant de l'eau stérile, l'eau stérile étant ajoutée à la composition pharmaceutique avant l'administration locale.

**7.** Un procédé pour la préparation d'une composition pharmaceutique selon la revendication 1, caractérisé en ce que les excipients sont mis en solution dans un solvant, la solution est absorbée sur le bromure d'otilonium et les solvants sont chassés par séchage.

**8.** Un procédé pour la préparation d'une composition pharmaceutique selon la revendication 1, caractérisé en ce que les excipients sont mélangés et fondus et les excipients fondue sont absorbée sur le bromure d'otilonium dans un appareil approprié.

**Revendications pour les Etats contractants suivants : ES, GR**

**1.** Procédé pour la préparation d'une composition pharmaceutique comprenant du bromure d'otilonium comme principe actif, caractérisé en ce que l'on ajoute audit bromure d'otilonium au moins un excipient antimousse et au moins un excipient émulsifiant.

**2.** Procédé selon la revendication 1, caractérisé en ce que l'excipient antimousse est un dérivé méthylique ou phénylique de polysiloxane.

**3.** Procédé selon la revendication 1 ou 2, caractérisé en ce que l'excipient émulsifiant est choisi parmi un ou plusieurs des suivants : un ester de sucre, un ester de sorbitan d'acides gras, un ester de polyoxyéthylènesorbitan d'acides gras, un ester polyoxyéthylénique d'acides gras, un èther polyoxyéthylénique d'alcools supérieurs, un ester d'acide gras.

**4.** Procédé selon l'une quelconque des revendications précédentes, caractérisé en ce que l'on utilise la formulation suivante :

| | |
|---|---|
| bromure d'otilonium | 10-300 mg |
| diméthylpolysiloxane | 0,3-10 mg |
| monostéarate de glycéryle | 0,01-0,5 mg |
| monopalmitate de sorbitan | 0,08-2,5 mg |
| monopalmitate de saccharose | 0,01-0,5 mg |
| eau | 12-18 ml |

**5.** Procédé selon la revendication 4, caractérisé en ce que l'on utilise la formulation suivante :

| | |
|---|---|
| bromure d'otilonium | 150 mg |
| diméthylpolysiloxane | 5 mg |
| monostéarate de glycéryle | 0,25 mg |
| monopalmitate de sorbitan | 1,25 mg |
| monopalmitate de saccharose | 0,25 mg |
| eau | 15 ml |

**6.** Procédé selon la revendication 1, caractérisé en ce que les excipients sont mis en solution dans un solvant, la solution est absorbée sur le bromure d'otilonium et les solvants sont chassés par séchage.

**7.** Procédé selon la revendication 1, caractérisé en ce que les excipients sont mélangés et fondus et les excipients fondus sont absorbés sur le bromure d'otilonium dans un appareil approprié.